(19) Europäisches Patentamt
European Patent Office
Office européen des brevets

(11) **EP 3 483 828 A1**

(12) **EUROPEAN PATENT APPLICATION**

(43) Date of publication:
**15.05.2019 Bulletin 2019/20**

(51) Int Cl.:
**G06Q 30/06** *(2012.01)*   **A61L 9/12** *(2006.01)*

(21) Application number: **17382765.0**

(22) Date of filing: **13.11.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Noustique Perfumes, S.L.**
**08002 Barcelona (ES)**

(72) Inventors:
• **Bui Tran, Duc Hanh**
  **81247 München (DE)**

• **Jedlinski, Jakub Jan**
  **85579 Neubiberg (PL)**
• **Lasala Alonso, Hugo**
  **50006 Zaragoza (ES)**
• **Suárez Iribarne, Alvaro**
  **80339 München (ES)**

(74) Representative: **ZBM Patents - Zea, Barlocci & Markvardsen**
**Plaza Catalunya, 1**
**08002 Barcelona (ES)**

(54) **COMPOSING A FRAGRANCE GOOD**

(57)    A method of composing a fragrance good (FG) from multiple essences ($E_1$-$E_{13}$) for subsequent dispensing by a dispensing appliance (1; 21), comprising: displaying, in a graphical user interface (TS), an essence map (EM) with several essences ($E_1$-$E_{13}$), receiving, from a user, a selection ($E_1$, $E_4$, $E_7$, $E_8$, $E_{10}$, $E_{13}$) of at least one displayed essence ($E_1$-$E_{13}$), and composing the fragrance good (FG) from the selected at least one essence ($E_1$-$E_{13}$), wherein the essence map (EM) comprises a coordinate space spanned by multiple different discrete finite axes (x, y), each axis is scaled by positive values according to a relative olfactory dominance in relation to at least one fragrance family, and wherein each essence ($E_1$-$E_{13}$) is attributed one value ($x_1$, $x_2$, $y_1$, $y_2$) on each axis (x, y). A dispensing system comprises at least one GUI (graphical user interface) device (SM) and at least one dispensing appliance (1; 21), the system being adapted to perform the method.

Fig.2

**Description**

The invention relates to a method of composing a fragrance good. The invention also relates to a dispensing system, comprising at least one GUI ("Graphical User Interface") device and at least one dispensing appliance, wherein the system is adapted to perform the method. The invention further relates to a computer program product for composing a fragrance good from multiple essences. The invention is particularly useful for composing a fragrance good in form of a fragrance blend or a cosmetic blend from multiple essences comprising at least one aroma compound.

US 2016/107187 A1 discloses a method, computer program product, and apparatus for receiving, at a fluid dispensing apparatus, a control signal, wherein the control signal may be received from a base station, wherein the control signal, when received, may cause the fluid dispensing apparatus to perform operations. The operations may include generating a positive pressure within a fluid cartridge of the fluid dispensing apparatus by adjusting, via a motor of the fluid dispensing apparatus, a drive rod and piston of the fluid dispensing apparatus in a first direction relative to the fluid cartridge to dispense fluid in the fluid cartridge via a nozzle of the fluid dispensing apparatus. The operations may include generating a negative pressure within the fluid cartridge by adjusting, via the motor, the drive rod and piston in a second direction relative to the fluid cartridge to draw fluid into the fluid cartridge via the nozzle.

NZ 607641 A discloses a container system for liquids such as spray fragrances. The system includes a parent container and a child container. The parent container provides a first cavity for confining a liquid, and couples detachably to the child container for refilling the child container through a supply opening in the parent container. The child container can be used for instance as a travel pack in a handbag or hand luggage. Here the child container is a compact dispenser, comprising a bowl-shaped rigid container having a refill opening, which is kept closed by a valve unless the dispenser is connected to the parent container, and a dispensing opening. These openings are located apart from each other. A pump dispenses liquid from the dispense opening. In a particularly simple construction, the opening of the bowl is covered by a deformable membrane to form a closed dispense cavity, the cavity becoming mainly or completely evacuated as the dispensing means is operated. The membrane then relaxes again, filling the dispenser, when the dispenser is re-applied to the parent container.

CN 202687926 U discloses a perfume filling machine, which comprises a support, a towing bracket arranged in the support, a filling frame arranged at the top of the support, more than one injection device arranged on the filling frame, and a perfume box arranged on one side of the support, wherein a pump body is arranged on the right side in the filling frame, the pump body is connected with the perfume box through a conduit, and the pump body is connected with more than one water pipe. According to the utility model, multiple perfumes can be filled simultaneously, so that the filling efficiency is improved.

CN 202400818 U discloses a valve provided with a pipeline and applicable for fragrance sliding way filling. The valve comprises a valve, wherein a handle switch is arranged on the valve; the vale also comprises a pipeline, wherein the valve is arranged on the pipeline; the valve is closed when the handle switch is parallel to the pipeline; and the valve is opened when the handle switch and the pipeline incline. The valve provided with the pipeline is simple in structure and convenient to operate; the accident of fragrance leakage resulted from misopening caused by the collision of the handle when sliding downwards along the sliding way; the environment pollution and the cost are reduced; and meanwhile, the pipeline and the valve are fixedly connected, so that the installation time is reduced, the production efficiency is improved greatly, and the production cost is saved.

US 6371451 B1 discloses a scent diffusion apparatus and method that contains individual original scents for a plurality of scents to be diffused, constructs information on position information of the contained original scents and information on various scents to be diffused in a database, and mixes the original scents of the desired scent properly at a desired point in time based on the scent information, to thereby spray the desired scent. The scent diffusion method includes providing a plurality of scent spraying units containing original scents, heating the original scents via a heater, evaporating the original scents, and diffusing the evaporated scent with the air via an air supply pump. Thus, a desired scent can be transferred to a selected subject under correct concentration. The scent diffusion apparatus uses scent cartridges containing the original scents, such as cartridges used in an existing ink-jet printer, and uses the same control commands as those of a general personal computer or ink-jet printer compatibly. Thus, the scent diffusion apparatus can control an amount of diffusion precisely, be manufactured in a compact fashion, and used in connection with a general-purpose computer in which almost all kinds of operating systems supporting an ink-jet printer are incorporated.

US 2012247613 A1 discloses a vending machine for dispensing a selected blend of perfumes in response to the deposit of a pre-selected sum of money includes a plurality of containers and a plurality of distinctive scents with one of the scents disposed in each of the containers. A mechanism for selecting one or more of the scents and an amount of one or more of the scents is provided. Further, the vending machine includes a mechanism for blending the selected scents in the selected percentages and providing a sample to a perspective customer. Further, the mechanism includes a slot receiving payment for a quantity of the selected blend together with a delivery process for providing a bottle of the selected perfume to the customer.

US 2016045838 A1 discloses an aroma substance delivery device adapted to deliver an aroma substance exclusively to the nasal cavities of an individual, comprising the following elements: (i) a source of carrier gas flow; (ii) regulating means which receives the carrier gas flow and regulates its passage through a plurality of channels; (iii) downstream of the regulating means a plurality of aroma substance-containing cartridges, one being associated with each channel, and (iv) disseminating means located in close proximity to the cartridges and adapted to deliver the individual aroma substances from the cartridges to nasal cavities by means of a conduit; there being positioned in each channel on the upstream side and in close proximity to the origin of the channel a flow restrictor, the flow restrictor being configured such that the pressure drop across the flow restrictor is higher than the pressure drop across the rest of the device. The device is particularly useful as an element in conjunction with an audio-visual display, to provide a complete sensory experience.

It is the **object** of the present invention to at least partially overcome the problems associated with the prior art. It is a particular object of the present invention to provide an easy-to-use means for composing a fragrance good.

The object is achieved according to the features of the independent claims. Advantageous embodiments can be found, e.g., in the dependent claims and/or in the description.

The object is achieved by a method of composing a fragrance good from multiple essences for subsequent dispensing by a dispensing appliance, the method comprising:

- displaying, in a graphical user interface of a GUI device, an essence map with several essences,
- receiving, from a user, a selection of at least one essence displayed in the essence map, and
- composing the fragrance good from the selected at least one essence,

wherein

- the essence map comprises a coordinate space spanned by multiple different discrete finite axes,
- each axis is scaled by positive values according to a relative olfactory dominance of an essence in relation to at least one fragrance family, and wherein
- each essence is attributed one value on each axis.

This method gives the advantage that the fragrance good can be composed in a particularly intuitive, clear and easy-to-use manner. In particular, a multitude of essences may be neatly arranged in the coordinate space in such a way that a user can intuitively link their positions to certain fragrance families.

The "fragrance good" may include a final fragrance blend and/or a final cosmetic product comprising at least one essence or final fragrance. A fragrance blend may be a Perfume (perfume extract, pure perfume), an Esprit de Parfum, an Eau de Parfum, an Eau de Toilette, an Eau de Cologne, or other type of fragrance blend etc. The method may include selecting the type of fragrance blend. The fragrance good may also be called a fragrance product.

The dispensing appliance may be a household appliance. This gives the advantage that a fragrance good may be composed by an end user.

The graphical user interface, GUI, is a component of the GUI device. The GUI device may be different from the dispensing appliance. For example, the GUI device may be a user's data processing device ("personal device"). The personal device may be a computer like a desktop computer or a laptop computer. The data processing device may also be a handheld device like a smartphone, a tablet computer etc. Alternatively or additionally, the dispensing appliance may be or comprise the GUI device.

In particular, the graphical user interface may be a touch screen. This enables an especially easy use of the essence map and further enhances a user experience.

An "essence" may comprise a mixture of one or more aroma compounds and one or more solvents. In particular, an essence may comprise one or more scents or olfactory components. In particular, an essence may correspond to the content of a container that can be stored in the dispensing appliance to be dispensed from the dispensing appliance.

The receiving of a selection of at least one essence displayed in the essence map may include receiving a user input at the GUI device corresponding to the at least one essence. For example, a user may touch or tap a position on a touch screen corresponding to a displayed position of an essence of the essence map. To this effect, the GUI device may be adapted to allow a user to select and deselect at least one essence shown in the essence map.

In particular, the essence map may be a graphical representation using a coordinate space or coordinate system of two or more axes. Each axis may have a discrete scale with a maximum value. In particular, each axis may be scaled by positive integer values that may include the value zero, e.g. 0, 1, 2, ..., 500.

The values of each axis describe relative dominance values of the essence in relation to at least one fragrance family. The specific relation to the at least one fragrance family for each axis may be called a "descriptor". Generally, the descriptor for one axis may comprise a description for one or for more fragrance families.

The descriptor may include a "more / less" description. For example, a value assigned to a certain essence may be the higher the more this essence is distinct or pronounced ("is dominated") with respect to a certain fragrance family. For example, if a certain essence is more "oriental" than another essence, it is assigned a higher value on a "oriental" axis. In analogy, a value assigned to a certain essence may be the lower the less this essence is distinct or pronounced ("is dominated") with respect to a certain fragrance family.

The descriptor may include two or more fragrance families for each axis. The descriptor for a certain axis may include a reverse relation for two fragrance families. For example, higher values may mean for an essence to be more pronounced regarding a first fragrance family (e.g. "oriental" or "floral") but less pronounced regarding a second fragrance family (e.g. "fresh" or "woody"), and vice versa.

The fragrance families may include first level fragrance families like "woody", "oriental", "fresh", and "floral" etc. The fragrance families may include second level fragrance families like "woods", "dry woods", "mossy woods", "woody oriental", "oriental", "soft oriental", "floral oriental", "soft floral", "floral", "fruity", "green", "water", "citrus", and "aromatic" etc.

It is an embodiment that each essence is attributed a radius (denoted by "r") reflecting a dilution value of pure aroma compounds of the essence. The radius reflects a "strongness" or "olfactory power" of this essence. This gives the advantage that the "strongness" or "olfactory power" of the essences can be considered when choosing or replacing essences while composing the fragrance good. In particular, the radius r may be calculated according to r = [100% - percentage of solvent(s)]. Thus, an essence with a very low content of aroma compound(s) may have a small radius while an essence comprising less diluted aroma compound(s) has a larger radius. Instead of r, a product (a . r) may be used. The factor "a" may be a proportionality factor which may be the same for each essence or which may be individually assigned to each essence. Generally, instead of the radius r, a diameter d = (2·r) or an area A = ($\pi \cdot r^2$) etc. may be used.

It is an embodiment that the radius is adjustable or selectable by a user. This gives the advantage that a strongness or olfactory power of this essence can be set by the user. To dispense the composed fragrance good, the dispensing appliance may dilute the respective essence according to the selected radius. To this effect, the dispensing appliance may comprise one or more dedicated solvent tanks.

It is an embodiment that the essence map comprises a coordinate space spanned by only two axes. This gives the user an especially good overview over the essences.

It is an embodiment that at least one axis is scaled in ascendance towards a more oriental fragrance and a less fresh fragrance. This gives the advantage that positioning of essences along this axis is particularly easy to grasp or understand.

It is an embodiment that at least one axis is scaled in ascendance towards a more floral fragrance and a less woody fragrance. This gives the advantage that positioning of essences along this axis is particularly easy to grasp or understand.

It is an embodiment that the essence map comprises a coordinate space spanned by two axes, wherein one axis is scaled in ascendance towards a more oriental fragrance and a less fresh fragrance and the other axis is scaled in ascendance towards a more floral fragrance and a less woody fragrance. This gives the advantage that the fragrance families "woody", "oriental", "fresh", and "floral" can be represented in an easy to understand fashion in a two-dimension representation.

In an embodiment, all potentially (in particular, commercially) available essences for a certain dispensing appliance or a certain type of dispensing appliances are displayable on the essence map. In a variant, only essences fitting one or more user-selected criteria are displayed on the essence map. The user-selected criteria may include: price, manufacturer, inclusion or exclusion of certain aroma compounds or fragrances, etc. This gives the advantage that visual clutter of the essence map (that could occur when a high number of essences exist) may be reduced.

It is an embodiment that the method comprises: highlighting essences from at least one pre-defined group of essences. This advantageously allows highlighting (e.g. by displaying in a different colour, a higher brightness etc.) certain essences in a multitude of essences.

It is an embodiment that the pre-defined group of essences comprises: essences belonging to at least one pre-defined fragrance good and/or essences available for a dispensing appliance. That the pre-defined group of essences comprises essences belonging to a pre-defined fragrance good gives the advantage that such pre-defined fragrance good is particularly easy to change for personal purposes. Essences belonging to a pre-defined fragrance good may include essences belonging to a certain fragrance blend or cosmetic article. That the pre-defined group of essences comprises essences available for a dispensing appliance gives the advantage that this supports composing fragrances that can be dispensed by a dispensing appliance without or with only small difficulty to have these essences available. Essences available for a dispensing appliance may include essences actually stored in a certain dispensing appliance; i.e., essences actually available to be dispensed, e.g. as defined by essence containers inserted into a certain dispensing appliance. Essences available for a dispensing appliance may also include essences potentially storable in a certain dispensing appliance. This may include essences that are commercially available for a certain type of dispensing appliance.

It is an embodiment that the step of receiving a selection comprises adding and/or removing at least one essence from a pre-defined group of essences. This embodiment may be advantageously used to create new fragrance goods by adding / eliminating / exchanging essences from a group of essences belonging to a pre-defined fragrance good.

It is an embodiment that the method comprises: calculating a distance between selected essences and, if the distance is smaller than a pre-defined threshold, issuing an information (in particular, a message) to the user. This gives the advantage that the user may be informed about the fact that a replacement or addition of one essence by another essence lying within this pre-defined threshold distance may not lead a significant difference of the fragrance good. The distance is a length in the coordinate space between positions assigned to different essences. The smaller the distance, the higher is an olfactory similarity between essences. Issuing the information may comprise sending out and/or displaying a message. The information may include a comment that exchanging an essence of a certain fragrance good with a very "near" essence may lead only to a small difference in the smell of the fragrance good (potentially depending also on the radii of near essences: similar radii give a smaller olfactory distinction of the fragrance good than significantly differing radii).

It is an embodiment that the method comprises: calculating a centre of all selected essences and, if a position of the centre is outside a predefined area on the essence map, issuing an information, particularly a message, to the user. This gives the advantage that a user is given information to achieve a well-balanced fragrance good. This embodiment recognizes the fact that a well-balanced blend typically comprises scents from all three levels of the scent pyramid (i.e., bottom, middle, and top) and/or scents from more than one or two scent families. The centre of selected essences of many well-balanced fragrance goods typically lies in a central area of the essence map. This is particularly true if one axis is scaled in ascendance towards a more oriental fragrance and a less fresh fragrance. The other axis may then be scaled in ascendance towards a more floral fragrance and a less woody fragrance. The coordinates $x_c$ and $y_c$ of a respective centre of the selected essences for an essence map having two axes x and y may be calculated in analogy to an arithmetic mean, e.g. as:

$$x_c = \frac{\sum_{i=1}^{n} x_i}{n}; \ y_c = \frac{\sum_{i=1}^{n} y_i}{n}$$

with i = 1, ..., n being n selected essences having respective coordinates $x_i$ and $y_i$.

It is an embodiment that the centre of the selected essences is calculated in analogy to a weighted arithmetic mean, e.g. according to:

$$x_c = \frac{\sum_{i=1}^{n}(w_i \cdot x_i)}{\sum_{i=1}^{n} w_i}; \ y_c = \frac{\sum_{i=1}^{n}(w_i \cdot y_i)}{\sum_{i=1}^{n} w_i}$$

with i = 1, ..., n selected essences. The weights may be being determined from the respective radii of the selected essences. This gives the advantage that the strongness of the essences can be considered. That the weights are determined from the respective radii of the selected essences may include that the weights are equal to the respective radii (i.e., $w_i = r_i$) or that the weights are calculated from the radii, e.g., as the diameter, the area belonging to a circle of that radius, etc.

It is an embodiment that the method comprises: checking whether the fragrance good comprises essences from all of different predefined sectors of the essence map, and if not, issuing an information to the user. This also gives the advantage that the user gets a hint towards composing a well-balanced fragrance good. The sectors may correspond to various levels or notes of the scent pyramid.

It is an embodiment that the information particularly includes presenting essences from the missing sectors. This gives the advantage that a user, if notified of essences missing from a certain sector, may immediately choose one or more presented essences from this sector. The presenting may include displaying, in the essence map, essences of this missing sector and/or potentially visually highlighting essences of this missing sector.

It is an embodiment that the method further comprises: transferring data regarding the composed fragrance good, including the selected essences, to the dispensing appliance; and receiving, by the dispensing appliance, the transferred data and dispensing the fragrance good based on the transferred data. In this way, the method of composing a fragrance good becomes a method of producing a fragrance good. The additional features give the advantage that a "recipe" for a fragrance good can be delivered to a dispensing appliance in a particularly convenient fashion to be dispensed from the dispensing appliance with only a low degree of further user interaction. The transferred data at least includes data regarding the selected essences (e.g., their ID, description etc.), and potentially: a name of the fragrance good, a date of creation, a creator's ID etc.

The transferring and receiving of the data may include performing wireless and/or wire-bound communication, e.g. via a network (e.g., the internet) or via direct communication. Wireless communication may include communication via telecommunication networks, e.g. using 3G, LTE; communication via a local area network like WLAN etc.; and/or direct communication like communication via Bluetooth. Wire-bound communication may include communication via Ethernet etc. The user interface and the dispensing appliance may be equipped with at least one respective communication means, like a WLAN module, an Ethernet module etc.

The object is also achieved by a dispensing system, comprising at least one GUI device and at least one dispensing appliance, wherein the system is adapted to perform the method as described above. The dispensing system can be embodied in analogy to the method and gives the same advantages.

It is an embodiment that the dispensing appliance is adapted to store several containers, each container comprising one essence. This advantageously facilitates dispensing fragrance goods based on the received data of the selection.

It is an embodiment that the dispensing appliance is adapted to transfer data to the GUI device, the data comprising information at least about the essences presently available in the dispensing appliance, i.e. their ID, potentially their current volume etc. This further facilitates composing fragrance goods based on presently available essences.

It is an embodiment that the system further comprises a database connectable to the GUI device in which database "essence data" data regarding the essences as such (e.g., their name, their content, their description etc.), their coordinates within the essence map and potentially their radius etc. are stored. This allows selecting and/or dispensing of essences and/or fragrance goods by dispensing appliances that basically can be positioned at any point in the world. It is an embodiment that essence data downloaded from the database onto a GUI device may be locally stored in the GUI device. This gives the advantage, that the composing of the fragrance good may be performed offline. Alternatively, to perform the method, the GUI device may be connected to the database via a network. In the following, mentioning the database may include an entity (e.g. a server, a cloud storage etc.) in which the database is integrated, if not described otherwise.

In one embodiment, communication between the GUI device in form of a personal device and the dispensing appliance is realised directly between the GUI device and the dispensing appliance. In another embodiment, communication between the GUI device in form of a personal device and the dispensing appliance is realised indirectly via the database.

The database may be part of a co-ordination entity or "operator". This co-ordination entity or "operator" may comprise or may be linked to a digital or online shop that sells essences. Particularly in this case, data regarding the essences actually / potentially available in the dispensing appliances may be stored in the database, e.g. in form of descriptions.

It is an embodiment that the system further comprises a machine learning entity performing machine learning on the data stored in the database, e.g. to find a user's olfactory preferences and to recommend essences to the user. This gives the advantage that preferences of one or more users regarding use of certain essences or kinds of essences and/or preferences regarding certain fragrance goods may be identified or even predicted.

It is an embodiment that - to perform the machine learning - data stored in the database are differentiated between predictor variables and predicted variables. In particular, the predicted variables may be or comprise "response data". The response data may comprise data concerning a rating / assessment / evaluation / feedback of the fragrance goods by at least one user. These data are consciously given by at least one user and are thus of particularly high relevance and prediction usefulness. The response data may comprise "likes" or "not likes" for resulting blends produced or to be produced the user, ratings of resulting blends produced or to be produced etc. The response data may be given by the user and eventually by other persons (e.g. other persons trying out the same blended fragrance good). Additionally, the data about "likes" and "not likes" may be indirectly retrieved from dispensing appliances where the feature "test blend" is enabled. When this feature is enabled, the user has the possibility to produce a small sample of fragrance goods, i.e., the test blend. In case the user does not like the sample, he/she will not produce an additional amount. In such a case, the recipe will be categorized into "not liked" by the specific user. Otherwise the user will produce an additional amount of the blend, which action will be automatically categorized to mean a "like" by the specific user.

The predictor variables may comprise any data concerning selections of the fragrance goods, including any data concerning the essences, e.g. the essence data. The selection data may also comprise data concerning an identity and/or a profile of a user choosing a certain fragrance good ("personal background data").

It is an embodiment that the predictor variables are grouped into matrices and the predicted variables are grouped into vectors. This further facilitates use of a statistical learning algorithm to prioritize presentation of fragrance goods on the graphical user interface for selection by the user.

Particularly, the matrices and vectors can be created for statistical supervised and unsupervised learning. Such statistical supervised and unsupervised learning is generally known in the art and is not explained further herein. Examples of supervised learning methods comprise linear methods such as thin plate spline or generalize additive models (GAM), or non-lineal methods such as support vector machines with non-lineal kernels. Supervised learning methods are particularly useful to train co-ordinating entity and a system comprising the co-ordinating entity, respectively, about predicting when a user will like a new blend, or not. Examples of unsupervised learning methods include K-means clustering or hierarchical clustering, which is particularly useful to find patterns and correlations between social profiles and liked or disliked recipes, and also to refine customer profiles by creating additional ones.

Particularly, the predictor variables (predictors) may be coded in the form of matrix components $x_{ij}$ and may represent the j-th value for the i-th observation corresponding to each predictor variable. The index i may have the values $I = \{1, 2, ..., n\}$ and the index j may have the values $j = \{1, 2, ... , p\}$. The index i may be used to index the samples or observations (from 1 to n) and the index j may be used to index the variable values of each predictor (from 1 to p). The matrix components $x_{ij}$ may

be grouped into a (n x p)-matrix X according to:

$$X = \begin{pmatrix} x_{11} & \cdots & x_{1p} \\ \vdots & \ddots & \vdots \\ x_{n1} & \cdots & x_{np} \end{pmatrix}$$

Response data obtained concerning or containing a user's feedback may be coded in the form of vector components $y_i$ to denote the i-th observation of the variable on which one wishes to make predictions. For example, to find a correlation between users and liked / not liked recipes, the variable to be predicted may have a value of 0 (not liked) or 1 (liked), and there will be i observations, namely one per every recipe a user has tested with the blending device. The vector components $y_i$ may, e.g., grouped in the following form:

$$\vec{y} = \begin{pmatrix} y_1 \\ \vdots \\ y_n \end{pmatrix}$$

Particularly after having all predictor variables and predicted variables coded into numbers in the form of matrixes and or vectors, the data may be cleaned, classified, integrated and aggregated for data analyses, interpretation and machine learning purposes.

Different matrices for distinctive features may be created using tools of statistical analysis in order to train the co-ordinating entity / the system with data and to test how predicted observations using the predictors (features) fit to real observations.

The dispensing appliance may be a blending device (also called a "fragrance blender") comprising a blending chamber for blending selected essences before dispensing the final blend / fragrance good. The dispensing appliance may be adapted to dispense the fragrance good into a receptacle, e.g. into a flacon.

It is an embodiment that the dispensing appliance comprises a cartridge rotatable around a rotational axis, the cartridge comprising multiple seats for containers; and a stem movable parallel to the rotational axis; wherein the cartridge is rotatable such that the seats are individually positionable in line with the stem.

It is an embodiment of the dispensing appliance that the cartridge comprises at least two levels individually rotatable around the rotational axis; wherein an upper level comprises an opening positioned in line with the stem and formed to let the stem pass through if the upper level is in a pre-determined rotational feed-through position; and wherein a lower level comprises an opening formed and positioned in line with the stem if the lower level is in a pre-determined rotational flow-through position.

It is an embodiment of the dispensing appliance that the levels are disk-shaped levels.

It is an embodiment of the dispensing appliance that the openings are radial slits.

It is an embodiment of the dispensing appliance that the cartridge is supported by freely rotatable rollers.

It is an embodiment of the dispensing appliance that the cartridge is rotatable by a drive wheel.

It is an embodiment of the dispensing appliance that the cartridge is removable from the dispensing appliance.

It is an embodiment of the dispensing appliance that the cartridge is removable in one piece, including a rotary axle.

It is an embodiment of the dispensing appliance that each level of the cartridge is removable from a rotary axle.

It is an embodiment of the dispensing appliance that containers seating in the seats are individually replaceable with the cartridge remaining in the dispensing appliance.

It is an embodiment of the dispensing appliance that the dispensing appliance further comprises at least one reader to identify containers seated in the seats. This gives the advantage that all essences present in the dispensing appliance are known and may be reported to the database and/or to an external GUI device. Additionally, the dispensing appliance may be adapted to determine a (residual) quantity (volume) of the essences stored in the containers. This gives the advantage that this quantity may be reported to the database and/or to an external GUI device. A user may decide to buy a new container, if the quantity is low.

It is an embodiment of the dispensing appliance that at least one seat is occupied by a respective elongated container that is aligned in parallel to the rotational axis, the at least one elongated container comprising: a hollow cylindrical body; a plunger inserted into the body, the plunger being movable along a longitudinal axis of the body by the stem; a plug fixedly positioned within the body at a longitudinal distance from the plunger such that the body, the plug and the plunger form a cavity; and a channel leading through the plug.

It is an embodiment of the dispensing appliance that the container further comprises a needle fluidly connected with the channel, a free end of the needle being positioned outside the cavity, and the needle being fully housed within the hollow cylindrical body.

The dispensing appliance may be operated such that at least one container is seated in a seat of the upper level and at least one container is seated in a seat of the lower level; the dispensing appliance is instructed to produce a blend from the content of at least two containers; the dispensing appliance rotates the upper level such that a corresponding container is positioned in line with the stem and rotates the lower level such that it is in its rotational flow-through position and / or the dispensing appliance rotates the upper level such that it is in its feed-through position and rotates the lower level such that a corresponding container is positioned in line with the stem; and the dispensing appliance then pushes the stem onto the container in line with the stem to release liquid essence from this container.

The object is further achieved by a computer program product for composing a fragrance good from multiple essences for subsequent dispensing by a dispensing appliance, said computer program product comprising program code which, when executed on a GUI device having a graphical user interface, enables the GUI device to:

- display, in the graphical user interface of the GUI device, at least one essence in an essence map;
- receive a selection of at least one essence displayed in the essence map; and
- compose the fragrance good from the selected at least one essence,

wherein

- the essence map comprises a coordinate space spanned by multiple different discrete finite axes,
- each axis is scaled by positive values according to a relative dominance in relation to at least one fragrance family, and wherein
- each essence is attributed one value on each axis.

The computer program product may be embodied in analogy to the method and/or to the dispensing appliance and gives the same advantages.

The computer program product may be embodied as one or more application programs or "apps" that are used to perform the method, e.g. on a smartphone, notebook, laptop, tablet etc.

The above described features may be freely combined to provide further embodiments. Features described with regard to one claim category (method, system, apparatus etc.) are applicable to all other claim categories (method, system, apparatus etc.) if not stated otherwise.

The above described features and advantages of the invention as well as their kind of implementation will now be schematically described in more detail by at least one embodiment in the context of one or more figures.

Fig.1 shows a system for dispensing a fragrance good comprising a GUI device having a touchscreen, a database, and a fragrance blender;

Fig.2 shows a generalized essence map displayed on the touchscreen;

Fig.3 shows the essence map with different essences;

Fig.4 shows an oblique view of a dispensing appliance comprising a cartridge according to a first embodiment;

Fig.5 shows an oblique view of an enlarged cut-out of the dispensing appliance of Fig.4;

Fig.6 shows a side view of an enlarged cut-out of the dispensing appliance of Fig.4;

Fig.7 shows an oblique view of the cartridge filled with the containers according of Fig.4;

Fig.8 shows an oblique view of a cartridge filled with containers according to a second embodiment of a dispensing appliance;

Fig.9 shows a top-down view of the cartridge of Fig.8;

Fig.10 shows an oblique view of one level of the cartridge of Fig.8 with a rotary axle; and

Fig.11 shows an oblique view of the one level of the cartridge of Fig.8 without the rotary axle.

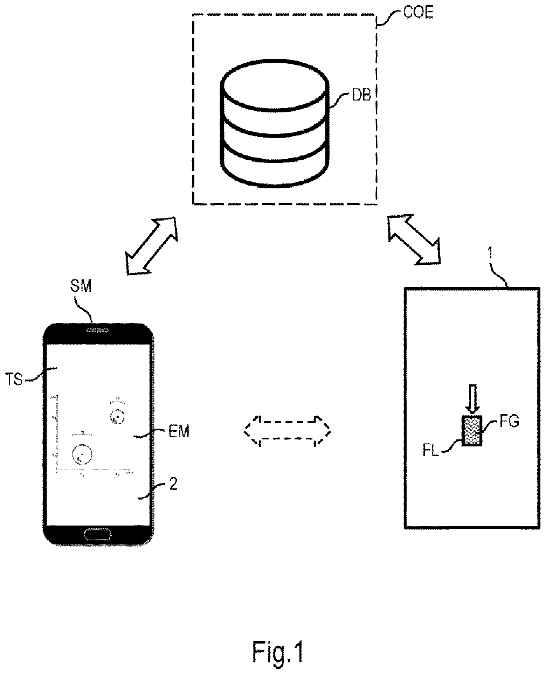

**Fig.1** shows a simplified system for dispensing a fragrance good FG, comprising at least one user's GUI device in form of a smartphone SM having a graphical user interface in form of a touchscreen TS, a database DB, and a dispensing appliance in form of a fragrance blender 1. The database DB may be part of a co-ordination entity COE.

The touchscreen TS may display an essence map EM. Essence data related to the essence map EM may be locally stored in the smartphone SM and/or may be stored in the database DB.

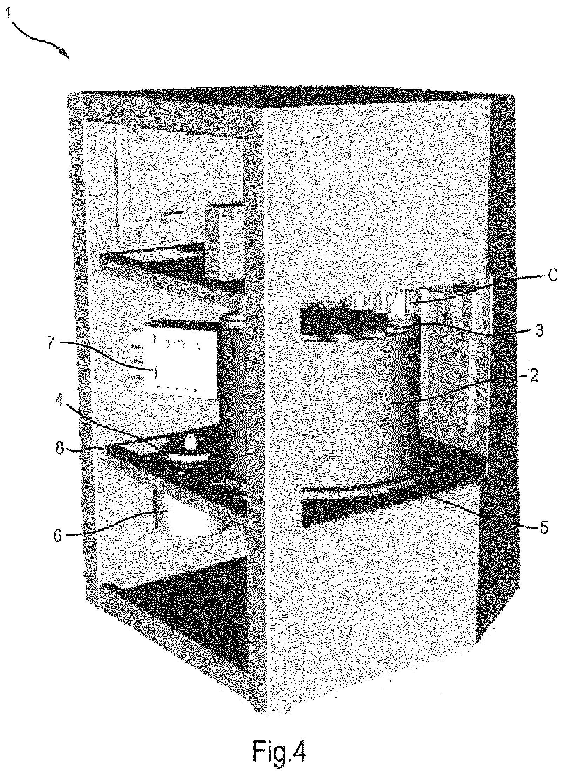

The smartphone SM can be communicatively coupled to the co-ordination entity COE and the database DB, respectively (as indicated by the respective double-sided arrow). The fragrance blender 1 can also be communicatively coupled to the co-ordination entity COE and the database DB, respectively (as indicated by the respective double-sided arrow). It is an option that fragrance blender 1 can be communicatively coupled to the smartphone SM (as indicated by the dashed double-sided arrow). The fragrance blender 1 is adapted to store several containers C (see Fig.4 ff. below) with each container C comprising one essence.

To compose a fragrance good FG from several essences C for subsequent blending and dispensing by the fragrance blender 1, a method is employed that comprises:

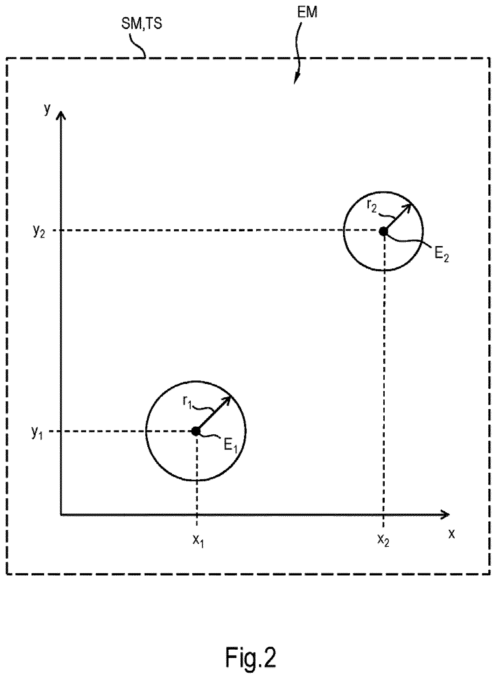

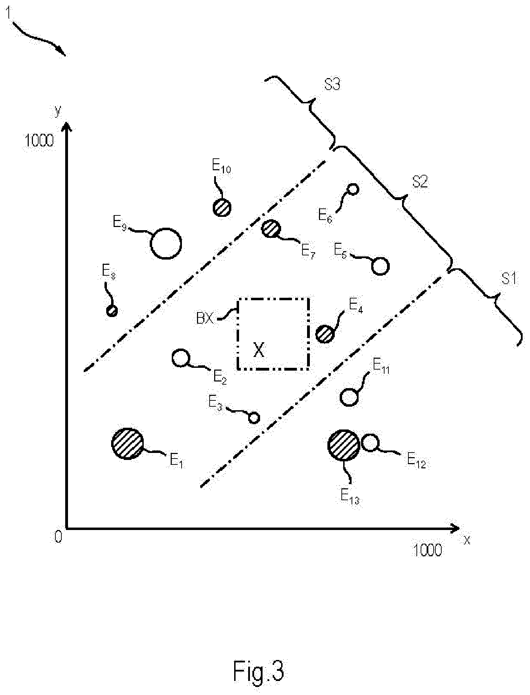

- displaying, on the touchscreen TS, the essence map EM with several essences $E_1$ to $E_{13}$ (see Fig.2 and Fig.3),
- the touchscreen TS receiving, from a user, a selection of at least one displayed essence $E_1$ to $E_{13}$, in the essence map, and
- the smartphone SM composing the fragrance good FG from the selected at least one essence $E_1$ to $E_{13}$.

Then, data regarding the composed fragrance good FG, including the selected essences E1 to E13, may be transferred to the fragrance blender 1. In one embodiment, these data are transferred via the co-ordination entity COE and database DB, respectively. Alternatively, the data are transferred directly to the fragrance blender 1.

After receiving the data, the fragrance blender 1 stores the data and may - e.g. prompted by a user - eventually blend and dispense a respective fragrance good FG, e.g. into a flacon FL or other receptacle.

**Fig.2** shows a generalized essence map EM displayed on the touchscreen TS of the smartphone SM showing two essences $E_1$ and $E_2$. The essence map EM has two axes, namely an x-axis x and a y-axis y. Thus, the essence map EM comprises a coordinate space spanned by the two axes x and y. In particular, the axis x is scaled in ascendance towards a more oriental fragrance and a less fresh fragrance. The axis y is scaled in ascendance towards a more floral fragrance and a less woody fragrance. Each axis x, y is scaled by positive integer values [0, 1, 2, ...] to a relative dominance of the essences $E_1$, $E_2$.

Each essence $E_1$, $E_2$ has been assigned a different position on the essence map EM comprising respective coordinates $(x_1, y_1)$ and $(x_2, y_2)$. The essence $E_1$ smells less oriental and fresher than essence $E_2$, as well as less floral and a woodier than essence $E_2$.

This assignment of the coordinates $(x_1, y_1)$ and $(x_2, y_2)$ may have been made by a user and/or by an expert perfumer or by a perfume chemist. An essence $E_1$, $E_2$ may have been assigned a respective position by different persons. In this case, the positions may be averaged to give one "compound" position.

Furthermore, each essence $E_1$, $E_2$ is attributed a radius $r_1$ and $r_2$, respectively, reflecting a dilution value of pure aroma compounds of the essences $E_1$, $E_2$. Since essence $E_1$ has a larger radius $r_1$ than essence $E_2$, it is less diluted and thus smells more intense than essence $E_2$.

**Fig.3** shows the essence map EM with different essences $E_1$ to $E_{13}$. The essences $E_1$ to $E_{13}$ may comprise all essences available for the fragrance blender 1, all essences present in the fragrance blender 1, or essences that have been pre-selected by a user according to certain criteria. A user may choose to highlighting essences $E_1$, $E_4$, $E_7$, $E_8$, $E_{10}$, and $E_{13}$ that belong to a predefined fragrance good FG.

A user may now touch or tap the bubbles representing the essences $E_1$ to $E_{13}$ to select (add) or deselect (remove) essences from the fragrance good FG. A user may also enlarge or reduce the radii of selected essences $E_1$, $E_4$, $E_7$, $E_8$, $E_{10}$, and $E_{13}$ to adjust their relative olfactory power. This may be done, e.g. by a two-finger pinching movement on the touchscreen TS.

If a user wants to add an essence $E_{12}$ to the group of already selected essences $E_1$, $E_4$, $E_7$, $E_8$, $E_{10}$, $E_{13}$, the smartphone SM may calculate respective distances between the already selected essences $E_1$, $E_4$, $E_7$, $E_8$, $E_{10}$, $E_{13}$ and the newly added essence $E_{12}$ and issue an information to the user that at least one the distances is smaller than a pre-defined threshold. In the shown example, the smartphone SM may inform the user that essence $E_{12}$ is rather close to essence $E_{13}$ such that adding essence $E_{12}$ may not greatly change the smell of the overall fragrance good FG, in particular, since the essence $E_{13}$ is stronger than the essence $E_{12}$. The user

may or may not react to this information and thus may or may not add the essence $E_{12}$.

The user may then choose to transfer the data concerning the present fragrance good FG to the database DB and/or to the fragrance blender 1, e.g. by pressing an "OK" button or touching a respective field on the touchscreen TS.

It is an embodiment that, prior to sending the data concerning the fragrance good FG, including the selected essences $E_1$, $E_4$, $E_7$, $E_8$, $E_{10}$, $E_{13}$ and their radii, the smartphone SM calculates a centre X of all selected essences $E_1$, $E_4$, $E_7$, $E_8$, $E_{10}$, $E_{13}$. If a position of the centre X is outside a predefined area BX on the essence map EM, a respective information or message is issued to the user. The user may or may not react to this information and thus may or may not send the data to the fragrance blender 1. If essences are added or removed, the centre X is recalculated. In a variant, the centre X and the predefined area BX are constantly shown on the essence map EM. The centre X may be calculated according to a geometric mean or a weighted geometric mean.

Furthermore, prior to sending the data concerning the fragrance good FG, the smartphone SM may check whether the fragrance good FD comprises selected essences $E_1$, $E_4$, $E_7$, $E_8$, $E_{10}$, $E_{13}$ from different predefined sectors S1 to S3 of the essence may EM. These sectors S1 to S3 may correspond to various levels of a generally known scent pyramid, e.g. by associating sector S1 with bottom (base) notes, sector S2 with middle (heart) notes, and sector S3 with top (head) notes. For example, essences $E_{11}$ to $E_{13}$ strongly associated with woody and oriental scent families typically belong to bottom notes while essences $E_8$ to $E_{10}$ strongly associated with fresh and floral scent families typically belong to top notes. If one or more of the sectors S1 to S3 does not comprise a selected essence $E_1$, $E_4$, $E_7$, $E_8$, $E_{10}$, $E_{13}$, the smartphone SM may issue an information to the user that the resulting fragrance good FG may not be well-balanced. This may include highlighting essences of missing sectors S1 to S3 on the touch screen TS.

In the smartphone SM, a computer program product may be stored comprising program code which, when executed on the smartphone SM, allows performing the above described method, steps and/or functions. The computer program product may be stored in form of an "app".

**Fig.4** shows an oblique view of a fragrance blender 1 comprising a cartridge 2 partially filled with cylindrical containers C according to a first embodiment. The fragrance blender 1 may be a household appliance.

The cartridge 2 is of a cylindrical form and rotatable around a rotational axis R (see Fig.3) that coincides with its longitudinal axis. The cartridge 2 and its rotational axis R, respectively, are vertically aligned within the fragrance blender 1.

The cartridge 2 comprises multiple holes that are aligned in parallel to the rotational axis and that serve as seats 3 for the containers C. The seats 3 are arranged in angular symmetry around the rotational axis, in particular having the same radial distance from the rotational axis R and a uniform angular distribution. The seats 3 are formed to hold the containers C in vertical alignment and at the same height. If the containers C have the same length, they stick out of the top of the cartridge 2 at the same height. In particular, all seats 3 may have the same form.

The cartridge 2 is rotatable around the rotational axis R by a drive wheel 4 that is frictionally engaged with a lateral circular surface of the cartridge 2, namely with a laterally protruding circular rim 5. The drive wheel 4 is driven by a motor 6, e.g. an electric stepping motor 6. The stepping motor 6 is operable to rotate the cartridge 2 such that the positions of the containers C are stepwise altered. This can also be described as a revolver-type rotation.

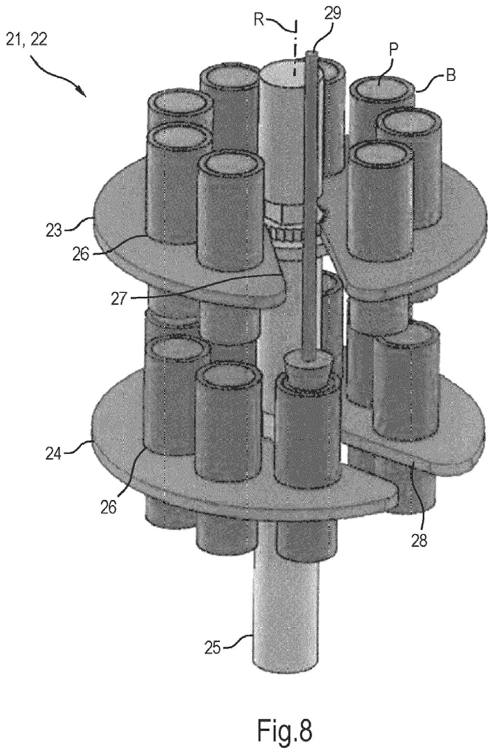

The fragrance blender 1 also comprises a stem 29 (see Fig.8) which may be linearly moved in parallel to the rotational axis R but has a fixed lateral or perpendicular position with respect to the rotational axis R. One of the seats 3 is in line with the stem 29 such that the stem 29 can be lowered towards this seat 3. If the seat 3 seats a container C, the stem may contact the container C to release a measured amount of essence from the container C. To achieve a precise dosage of the essence, the stem 29 is movable and/or is rotatable in a continuous or quasi-continuous manner.

By rotating the cartridge 2, one or more containers C are selectable to release essence, particularly from their bottom side. Thus, the cartridge 2 is rotatable such that the seats 3 are individually positionable in line with the stem 29.

The fragrance blender 1 further comprises a reader 7 (e.g., a barcode reader) to identify the containers C and/or their ingredient(s). The reader 7 may be coupled to a control unit (not shown) of the fragrance blender 1.

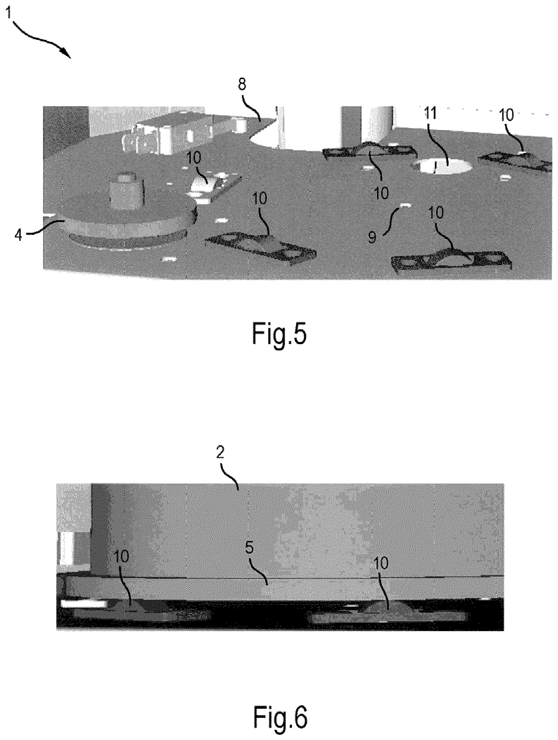

**Fig.5** shows an oblique view of an enlarged cut-out of the fragrance blender 1. The cut-out shows a compartment floor 8 that has a hole 9 for inserting a respective pin of a rotary axle of the cartridge 2 or of a bracket or frame thereof. Placed in radial symmetry around this hole 9 is a set of freely rotatable rollers in form of wheels 10. The wheels 10 are adapted to support the bottom surface of the cartridge, as can be seen in **Fig.6.**

Fig.5 also shows a through-hole 11 through which liquid released from a container C may fall or flow, e.g. into a blending chamber (not shown).

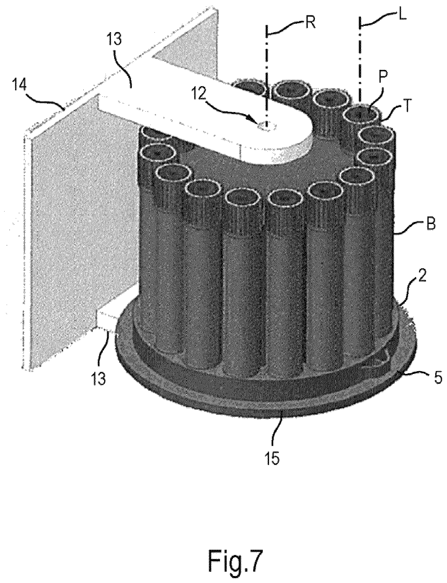

**Fig.7** shows an oblique view of the cartridge 2 filled with the containers C while a lateral side wall is not shown. A rotary axle 12 of the cartridge 2 rotational around the rotational axis L is inserted into a pair of arms 13. The cartridge 2 is freely rotatable with respect to the arms 13.

The arms 13 are held together by a cover 14. The arms 13 and the cover 14 act as a frame for the rotary axle 12. The cover 14 is used to close a service opening (not shown) of the fragrance blender 1. The lower arm 13 has a pin (not shown) at its lower side that can be inserted into the hole 9 for improved positional accuracy. When the cover 14 is removed, the cartridge 2 is removed with is, including the rotary axle 12. This facilitates replacement of the containers C.

Replacement of the containers C may include removing the cartridge 2 from the fragrance blender 1 and re-inserting the cartridge 2 into the fragrance blender 1. The replacement of the containers C may be performed in one or more of several ways:

In one variant, the complete unit including the cover 14, the arms 13, the cartridge 2, and the containers C is replaced.

In another variant, the cartridge 2 is disengaged from the arms 13 and is replaced together with the containers C.

In yet another variant, the cartridge 2 may or may not remain engaged with the arms 13, and a user replaces the containers C, in particular individually. Particularly in this variant, the user may detach the lateral side wall to replace the containers C. The containers C are then only seated in seats of a disk-shaped base 15 of the cartridge 2.

In all these variants, the cartridge 2 is removed from the fragrance blender 1 in one piece.

In even another variant (not shown), only the cartridge 2 and the containers C are removable from the fragrance blender 1 for replacement of the containers C.

Alternatively, the containers C are individually replaceable with the cartridge 2 remaining in the dispensing appliance (not shown). This may be facilitated by using a tap located in the middle of cover 14 such that the cartridge 2 may be rotated accordingly to put an empty container C at the tap.

The containers C are aligned in parallel to the rotational axis L. The containers C comprise a hollow cylindrical body B; a plunger P inserted into the body B, the plunger P being movable along a longitudinal axis L of the body B by the stem. For new containers C, the plunger P is positioned at or near the top face of the body B. The containers C further comprise a plug (not shown) fixedly positioned within the body B at a longitudinal distance from the plunger P such that the body B, the plug and the plunger P form a cavity (not shown); and a channel (not shown) leading through the plug towards a bottom face of the body B. The cavity is filled with liquid essence to be used in blending a fragrance blend. The fragrance blend may be a perfume, an Eau de Cologne etc., as chosen by the user. On the outside of its top section T, the body B is structured (here: dentate) to allow a safe grip by a user to handle the containers C.

**Fig.8** shows an oblique view of a cartridge 22 of a dispensing appliance 21 filled with containers C. The containers C may be the same containers as described for the first embodiment.

The cartridge 22 comprises two sections or levels 23 and 24 positioned one above the other. The levels 23 and 24 are individually rotatable around the rotation axis R. To this effect, the levels 23 and 24 may be rotatable around the same rotary axle 25 or around two collinear rotary axles. In the present embodiment, the configuration having only one rotary axle 25 is shown. This rotary axle 25 may have two or more sections that are freely rotatable around the rotation axis R.

The levels 23 and 24 may be rotatable by respective motors, e.g. via respective drive wheels (not shown).

Both levels 23, 24 comprise multiple (here: eight) seats 26 in form of holes into which the containers C are insertable. The seats are uniformly distributed around the rotational axis R. The seats 26 and the containers C may be formed such that a stop is provided to position the containers C at a pre-defined height with respect to the levels 23, 24. Both levels 23, 24 further comprise openings in form of linear radial slits 27 and 28, respectively. Both levels 23, 24 are basically disk-shaped and may be identical in shape.

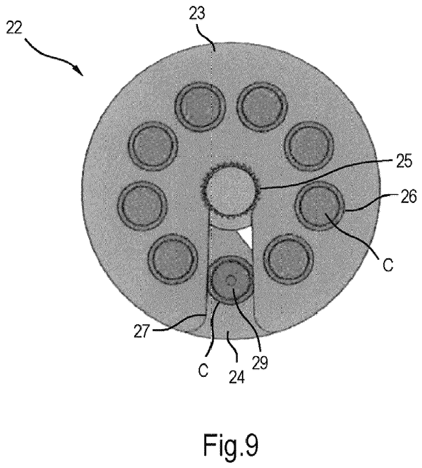

To release an essence from a container C seated in a seat 26 of the lower level 24, the dispensing appliance 21 rotates the upper level 23 such that it is in its feed-through position in which the upper slit 27 is in line with the stem 29. Thus, the stem 29 may pass through the upper slit 27. Furthermore, the dispensing appliance 21 rotates the lower level 24 such that a desired container C is in line with the stem 29. The dispensing appliance 21 then pushes the stem 29 onto this container C in line with the stem 29, e.g. to push its plunger P into its body B to release essence from this container C, as shown. **Fig.9** shows a top-down view of the cartridge 22 in this position.

To release an essence from a container C seated in a seat 26 of the upper level 23, the dispensing appliance 21 rotates the upper level 23 such that a corresponding container C is positioned in line with the stem 29 and rotates the lower level 24 such that it is in its rotational flow-through position in which the lower slit 28 is in line with the stem 29 and thus below the container C to be actuated (not shown).

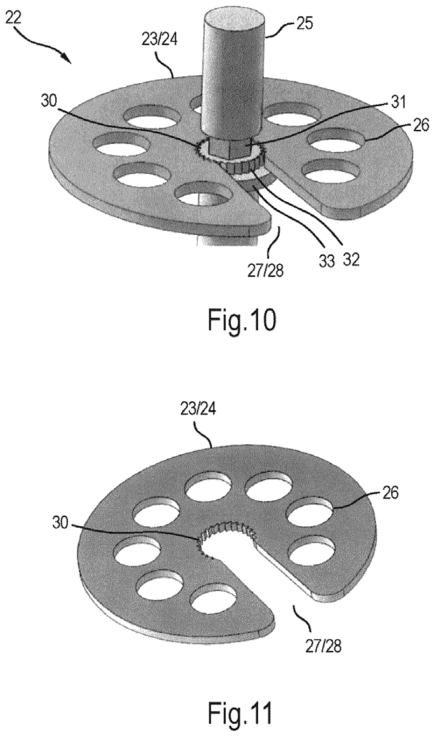

**Fig.10** shows an oblique view of one level 23 or 24 of the cartridge 22 with the rotary axle 25. **Fig.11** shows an oblique view of the one level 23 or 24 without the rotation axis.

To fix the levels 23 and 24 of the cartridge 22 to the rotary axle 25, the levels 23 and 24 have a central hole 30 that is dentate. The slits 27 and 28, respectively, connect the central hole 30 with the outer rim of the levels 23 and 24, respectively.

To attach the levels 23 and 24 to the rotary axle 25, the rotary axle 25 comprises a butted section 31 of smaller diameter than the slits 27, 28. Below the butted section 31 is a dented section 32 of the axle 25 that is formed to closely fit into the central hole 30. The engaging teeth of the central hole 30 and of the dented section 32 prevent rotation of the levels 23 and 24 around the axle 25.

Below the dented section 32 is a laterally extending flange 33 that acts as a support and longitudinal stop for the levels 23, 24.

To remove a level 23, 24 of the cartridge 22 from the rotary axle 25 (e.g. to replace containers C), a user may first lift the level(s) 23, 24 to the respective butted section 31 and then slide it off the axle 25 though the slit 27, 28. To insert the level 23, 24, the reverse steps can be performed.

Of course, the invention is not restricted to the described embodiments.

## LIST OF REFERENCE SIGNS

| | |
|---|---|
| 1 | Dispensing appliance |
| 2 | Cartridge |
| 3 | Seat |
| 4 | Drive wheel |
| 5 | Rim |
| 6 | Motor |
| 7 | Reader |
| 8 | Compartment floor |
| 9 | Hole |
| 10 | Wheel |
| 11 | Through-hole |
| 12 | Rotary axle |
| 13 | Arm |
| 14 | Cover |
| 15 | Base |
| 21 | Dispensing appliance |
| 22 | Cartridge |
| 23 | Upper level |
| 24 | Lower level |
| 25 | Rotary axle |
| 26 | Seat |
| 27 | Upper radial slit |
| 28 | Lower radial slit |
| 29 | Stem |
| 30 | Central hole |
| B | Body of the container |
| BX | Central area |
| C | Container |
| CO | Coordination entity |
| DB | Database |
| $E_1$-$E_{13}$ | Essences |
| EM | Essence Map |
| P | Plunger of the container |
| R | Rotational axis |
| L | Longitudinal axis of the container |
| $r_1$, $r_2$ | Radius |
| SM | Smartphone |
| TS | Touchscreen |
| x | X-axis of the essence map |
| $x_1$, $x_2$ | Coordinate of the x-axis |
| y | Y-axis of the essence map |
| $y_1$, $y_2$ | Coordinate of the y-axis |

## Claims

1. A method of composing a fragrance good (FG) from multiple essences ($E_1$-$E_{13}$) for subsequent dispensing by a dispensing appliance (1; 21), the method comprising:

   - displaying, in a graphical user interface (TS), an essence map (EM) with several essences ($E_1$-$E_{13}$),
   - receiving, from a user, a selection ($E_1$, $E_4$, $E_7$, $E_8$, $E_{10}$, $E_{13}$) of at least one displayed essence ($E_1$-$E_{13}$), and
   - composing the fragrance good (FG) from the selected at least one essence ($E_1$, $E_4$, $E_7$, $E_8$, $E_{10}$, $E_{13}$),

   wherein

   - the essence map (EM) comprises a coordinate space spanned by multiple different discrete finite axes (x, y),
   - each axis (x, y) is scaled by positive values according to a relative olfactory dominance in relation to at least one fragrance family, and wherein
   - each essence ($E_1$-$E_{13}$) is attributed one value ($x_1$, $x_2$, $y_1$, $y_2$) on each axis (x, y).

2. The method according to claim 1, wherein each essence ($E_1$-$E_{13}$) is attributed a radius ($r_1$, $r_2$) reflecting a dilution value of pure aroma compounds of the essence ($E_1$-$E_{13}$).

3. The method according to any of the preceding claims, wherein the essence map (EM) comprises a coordinate space spanned by two axes (x, y).

4. The method according to any of the preceding claims, wherein at least one axis (x) is scaled in ascendance towards a more oriental fragrance and a less fresh fragrance.

5. The method according to any of the preceding claims, wherein at least one axis (y) is scaled in ascendance towards a more floral fragrance and a less woody fragrance.

6. The method according to any of the preceding claims, wherein the method comprises:

   - highlighting essences ($E_1$, $E_4$, $E_7$, $E_8$, $E_{10}$, $E_{13}$) from at least one predefined group of essences.

7. The method according to claim 6, wherein the predefined group of essences ($E_1$, $E_4$, $E_7$, $E_8$, $E_{10}$, $E_{13}$) comprises:

   - essences belonging to at least one predefined fragrance good (FG) and/or
   - essences available for a dispensing appliance.

8. The method according to any of the claims 6 or 7, wherein the step of receiving a selection comprises adding and/or removing at least one essence from a pre-defined group of essences ($E_1$, $E_4$, $E_7$, $E_8$, $E_{10}$, $E_{13}$).

9. The method according to any of the preceding claims, wherein the method comprises:

   - calculating a distance between selected essences ($E_1$, $E_4$, $E_7$, $E_8$, $E_{10}$, $E_{13}$) and,
   - if the distance is smaller than a pre-defined threshold, issuing an information to the user.

10. The method according to any of the preceding claims, wherein the method comprises:

    - calculating a centre (X) of all selected essences ($E_1$, $E_4$, $E_7$, $E_8$, $E_{10}$, $E_{13}$) and,
    - if a position of the centre (X) is outside a predefined area (BX) on the essence map (EM), issuing an information to the user.

11. The method according to claims 2 and 10, wherein the centre (X) is calculated as a weighted centre with the weights being determined from the respective radii (r) of the selected essences ($E_1$, $E_4$, $E_7$, $E_8$, $E_{10}$, $E_{13}$).

12. The method according to any of the preceding claims, wherein the method comprises:

    - checking whether the fragrance good (FG) comprises essences from all of different predefined sectors (S1, S2, S3) of the essence map (EM), and
    - if not, issuing an information to the user, the information particularly including presenting essences of missing sectors.

13. The method according to any of the preceding claims, wherein the method further comprises:

    - transferring data regarding the composed fragrance good (FG), including the selected essences ($E_1$, $E_4$, $E_7$, $E_8$, $E_{10}$, $E_{13}$), to the dispensing appliance (1; 21); and
    - receiving, by the dispensing appliance (1; 21), the transferred data and dispensing the fragrance good (FG) based on the transferred data.

14. A dispensing system, comprising at least one GUI device (SM) and at least one dispensing appliance (1; 21), wherein

- the system is adapted to perform the method according to any of the preceding claims, and wherein
- the dispensing appliance (1; 21) is adapted to store several containers (2), each container (2) comprising one essence ($E_1$ - $E_{13}$).

15. The dispensing system according to claim 14 or 15, wherein the dispensing appliance (1; 21) is adapted to transfer data to the GUI device (SM, TS), the data comprising information at least about the essences ($E_1$ - $E_{13}$) presently available in the dispensing appliance (1; 21).

16. The dispensing system according to any of the claims 14 to 15, wherein the system further comprises a database (CO, DB) connectable to the GUI device (SM, TS) in which database (CO, DB) data regarding the essences ($E_1$ - $E_{13}$), their coordinates ($x_1$, $x_2$, $y_1$, $y_2$) and potentially their radius ($r_1$, $r_2$) are stored.

17. A computer program product for composing a fragrance good (FG) from multiple essences ($E_1$ - $E_{13}$) for subsequent dispensing by a dispensing appliance (1; 21), said computer program product comprising program code which, when executed on a GUI device (SM) having a graphical user interface (TS), enables the GUI device (SM) to:

- display, in the graphical user interface (TS), at least one essence ($E_1$ - $E_{13}$) in an essence map (EM);
- receive a selection of at least one essence ($E_1$, $E_4$, $E_7$, $E_8$, $E_{10}$, $E_{13}$) displayed in the essence map (EM); and
- compose the fragrance good (FG) from the selected at least one essence ($E_1$, $E_4$, $E_7$, $E_8$, $E_{10}$, $E_{13}$),

wherein

- the essence map (EM) comprises a coordinate space spanned by multiple different discrete finite axes (x, y),
- each axis (x, y) is scaled by positive values according to a relative dominance in relation to at least one fragrance family, and wherein
- each essence ($E_1$ - $E_{13}$) is attributed one value ($x_1$, $x_2$, $y_1$, $y_2$) on each axis (x, y).

Fig.1

Fig.2

Fig.3

Fig.4

Fig.5

Fig.6

Fig.7

Fig.8

Fig.9

22
25
23/24
30
31
26
27/28
33 32

Fig.10

23/24
26
30
27/28

Fig.11

Europäisches
Patentamt

European
Patent Office

Office européen
des brevets

## EUROPEAN SEARCH REPORT

Application Number

EP 17 38 2765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/018528 A1 (SAMAIN HENRI [FR]) 26 January 2012 (2012-01-26) * page 1, paragraphs 6-11, 16 * * page 2, paragraph 56 * * page 3, paragraph 67 * * page 6, paragraph 188 - paragraph 193 * * page 7, paragraphs 201-209, 212-216 * * page 8, paragraph 261 - paragraph 263 * * page 10, paragraph 310-316 * * page 11, paragraph 346 - paragraph 347 * ----- | 1-17 | INV. G06Q30/06 A61L9/12 |
| X | WO 2009/016590 A2 (OREAL [FR]; DURU NICOLAS [FR]) 5 February 2009 (2009-02-05) * page 20, line 16 - page 21, line 8 * ----- | 1-17 | |
| X | WO 2016/087467 A1 (OREAL [FR]) 9 June 2016 (2016-06-09) * page 39, line 5 - page 41, line 6 * * figures 31-37 * ----- | 1-17 | |
| | | | **TECHNICAL FIELDS SEARCHED (IPC)** G06Q A61L |

The present search report has been drawn up for all claims

| Place of search | Date of completion of the search | Examiner |
|---|---|---|
| The Hague | 21 February 2018 | Arbutina, Ljiljana |

EPO FORM 1503 03.82 (P04C01)

## ANNEX TO THE EUROPEAN SEARCH REPORT
## ON EUROPEAN PATENT APPLICATION NO.

EP 17 38 2765

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2018

| Patent document cited in search report | | | Publication date | Patent family member(s) | | | Publication date |
|---|---|---|---|---|---|---|---|
| US | 2012018528 | A1 | 26-01-2012 | CN | 102223901 | A | 19-10-2011 |
| | | | | EP | 2349356 | A1 | 03-08-2011 |
| | | | | FR | 2939001 | A1 | 28-05-2010 |
| | | | | JP | 2012510103 | A | 26-04-2012 |
| | | | | KR | 20110077013 | A | 06-07-2011 |
| | | | | US | 2012018528 | A1 | 26-01-2012 |
| | | | | WO | 2010058380 | A1 | 27-05-2010 |
| WO | 2009016590 | A2 | 05-02-2009 | FR | 2919505 | A1 | 06-02-2009 |
| | | | | WO | 2009016590 | A2 | 05-02-2009 |
| WO | 2016087467 | A1 | 09-06-2016 | CN | 106998884 | A | 01-08-2017 |
| | | | | EP | 3226718 | A1 | 11-10-2017 |
| | | | | JP | 2018500974 | A | 18-01-2018 |
| | | | | KR | 20170091137 | A | 08-08-2017 |
| | | | | US | 2017360178 | A1 | 21-12-2017 |
| | | | | WO | 2016087467 | A1 | 09-06-2016 |

EPO FORM P0459

For more details about this annex : see Official Journal of the European Patent Office, No. 12/82

**EP 3 483 828 A1**

## REFERENCES CITED IN THE DESCRIPTION

**Patent documents cited in the description**

- US 2016107187 A1
- NZ 607641 A
- CN 202687926 U
- CN 202400818 U
- US 6371451 B1
- US 2012247613 A1
- US 2016045838 A1